**Europäisches Patentamt**

**European Patent Office**

**Office européen des brevets**

(19)

(11) Veröffentlichungsnummer: **0 221 007**
**B1**

# (12) EUROPÄISCHE PATENTSCHRIFT

(45) Veröffentlichungstag der Patentschrift: **27.12.90**

(51) Int. Cl.⁵: **A 61 B 10/00**

(21) Anmeldenummer: **86730179.8**

(22) Anmeldetag: **30.10.86**

(54) Feinnadel-Biopsiekanüle mit Mandrin.

(30) Priorität: **30.10.85 DE 3538956**

(43) Veröffentlichungstag der Anmeldung:
**06.05.87 Patentblatt 87/19**

(45) Bekanntmachung des Hinweises auf die
Patenterteilung:
**27.12.90 Patentblatt 90/52**

(84) Benannte Vertragsstaaten:
**BE DE ES FR GB NL SE**

(56) Entgegenhaltungen:
**EP-A-0 019 104**
**EP-A-0 173 653**
**EP-A-0 186 256**
**DE-A-1 817 555**
**DE-A-3 026 657**
**JP-A-5 652 575**
**US-A-3 788 119**

**Die Akte enthält technische Angaben, die nach
dem Eingang der Anmeldung eingereicht
wurden und die nicht in dieser Patentschrift
enthalten sind.**

(73) Patentinhaber: **Lübbers, Heiko, Dr.**
**Hohnstorfer Strasse 84**
**D-3119 Bienenbüttel (DE)**

(72) Erfinder: **Lübbers, Heiko, Dr.**
**Hohnstorfer Strasse 84**
**D-3119 Bienenbüttel (DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1 (DE)**

**Beschreibung**

Die Erfindung betrifft ein Biopsiebesteck der im Oberbegriff des Patentanspruchs 1 angegebenen Art.

Bei Biopsiebestecken, die dazu dienen Gewebeproben aus dem menschlichen Körper zu entnehmen, unterscheidet man zwischen solchen, bei denen der in der Kanüle verschiebbare Mandrin einen Entnahmeraum aufweist, der durch das Vorschieben der Kanüle geschlossen wird, und solchen, bei denen das Innere der Kanüle als Entnahmeraum dient und bei denen der Mandrin keine Aussparung aufweist. Bei der zweiten Kategorie, auf die sich die Erfindung bezieht, wird nach erfolgter Punktion die Kanüle zur Probenahme über den Mandrin hinaus vorgeschoben. Hierbei muß die Kanüle eine schneidende Wirkung ausüben, ohne einen zu großen Biopsiedruck zu erzeugen. Es kommt also darauf an, daß die Kanüle ohne große Kraftanwendung in das zu untersuchende Organ eindringt.

Das aus JP—56—52575 bekannte Biopsiebesteck der zweiten Kategorie weist eine symmetrische volle Spitze auf, die das Gewebe lediglich auseinanderdrückt, aber keine schneidende Wirkung ausübt. Der Kanülenschliff ist ein Menghini-Schliff, der aus einer Kombination eines Schräganschliffs mit einem kegelförmigen Anschliff besteht. Ein solcher Kanülenschliff weist keine ausgeprägte Spitze auf und er dringt relativ stumpf in das Gewebe ein und erfordert eine hohe Biopsiekraft mit der Folge, daß der Patient Schmerz erleidet. Dies führt zu unkontrollierten Abwehrbewegungen, zu erhyblichen Zwerchfell-Exkursionen durch heftiges Durchatmen und im ungünstigsten Fall z.B. zu einem Kapseleinschnitt der Leber mit bedrohlichem Blutverlust.

Aus DE—PS 1 817 555 ist ein Biopsiebesteck der ersten Kategorie bekannt, bei dem der Mandrin im Abstand hinter der Mandrinspitze einen Entnahmeraum aufweist. Die Kanüle hat einen Menghini-Schliff, der facettenlos ist und ein abgerundetes Kanülenende bildet. Der Mandrin hat einen schrägen Hauptschliff mit Facetten zur Bildung einer ausgeprägten Mandrinspitze. Die Hauptschliffe von Kanüle und Mandrin haben unterschiedliche Orientierungen. Die Kanüle kann und soll nicht über die Mandrinspitze hinaus vorgeschoben werden. Würde sie vorgeschoben werden, dann könnte mangels einer scharfen Kanülenspitze kein atraumatischer Einstich ausgeführt werden.

Der Erfindung liegt die Aufgabe zugrunde, ein Biopsiebesteck der im Oberbegriff des Patentanspruchs 1 angegebenen Art derart weiterzubilden, daß beim Vorschieben der Kanüle über den Mandrin hinaus eine schnelle, atraumatische und wenig gewebszerstörende Biopsie ermöglicht wird. Dabei soll das teilweise beobachtete bisherige Zerfetzen von Zellen im Randbezirk der Biopsiekanüle, das eine Verschlechterung der Ausbeute bedeutet, ebenso vermieden werden wie aus Ausweichbewegungen vom Zielgebiet bei Punktionen an locker beweglichen Organen.

Die Lösung dieser Aufgabe erfolgt erfindungsgemäß mit den im kennzeichnenden Teil des Patentanspruchs 1 angegebenen Merkmalen.

Durch die kurz hintereinander folgende Anordnung beider Schliffe entsteht eine funktionelle Einheit zur Penetrations ins Zielorgan, die leicht, ohne Schmerzen für den Patienten und ohne das vorherige Abschneiden von falschem Gewebe abläuft.

Die eigentliche Gewebsbiopsie, die dann im Zielorgan stattfindet, geschieht mit zurückgezogenem Mandrin, wobei jetzt die beiden scharfen Facetten des Menghini-Schliffs zusammen mit der umlaugenden Schneidkante eine schnelle und wenig gewebszerstörende Biopsie erlauben.

Üblicherweise wird eine derartige Biopsie unter Zuhilfenahme von Ultraschall durchgeführt. Der untersuchende Arzt hält dabei in einer Hand den Schallkopf des Ultraschallgeräts und verfolgt auf einem Monitor das zu untersuchende Zielgebiet. Mit der anderen Hand kann die an einem Spritzenhalter adaptierte Aspirationsspritze mit der erfindungsgemäßen Biopsiekanüle in das Zielgebiet geführt werden.

Die Mandrinspitze soll dabei im Ultraschall gut sichtbar sein und eine gute Richtungstreue aufweisen um zu gewährleisten, daß auch tatsächlich das anvisierte Zielgebiet getroffen wird. Dazu wird der Mandrin zurückgezogen und die Kanüle kurz in das Zielgebiet vorgestoßen. Durch den mit Hinterschliff-Facetten geschärften Menghini-Schliff der Kanüle, ggf. in Verbindung mit Vakuum, das durch das Zurückziehen des Mandrins und des Kolbens in der Kanüle erzeugt, wird ein Gewebezylinder gewonnen. Infolge der verbesserten Schneideigenschaften der Kanüle können auch noch bei sehr kleinen Innendurchmessern zusammenhängende Gewebszylinder gewonnen werden. Mit herkömmlichen Schliffen würde hier nur noch eine Zellschmiere gewonnen werden.

Während der Biopsie sollte der Patient die Luft anhalten, um innere Verletzungen zu vermeiden. Da eine Biopsie mit der vorgeschlagenen Feinnadel-Biopsiekanüle nur ca. 1 bis 3 Sekunden dauert, ist ein ruhiges Verhalten des Patienten problemlos möglich.

Die ausgestantzte Probe kann außerhalb des Körpers durch das Verschieben des Mandrins ausgestrichen werden. Dabei wird diese Probe nicht zerstört und es wird keine mit Fremdgewebe durchsetzte Zellprobe sondern ein größerer Gewebezylinder gewonnen, der ausschließlich aus Gewebe des Zielgebiets besteht. Eine solche Gewebeprobe kann histologisch beurteilt werden.

Durch die Biopsie mit einem Kanülendurchmesser im Feinnadelbereich wird die Gefahr des Traumas erheblich verringert, der Patient hat weniger Schmerzempfinden, die Folgereaktionen des vegetativen Nervensystems werden verringert, ebenso die Schmerzabwehrbewegungen des Patienten.

Durch die Gleichsinnigkeit der spitzen Mandrin- und Kanülen schräganschliffe, die durch eine Verdrehsicherung sichergestellt ist, wird gewährleistet, daß der Mandrin durch das Gewebe eine Bahn vorschneidet, in der die nachfolgende Stanzkanüle geführt wird. Durch diesen Schnitt kann das Gewebe sich nicht so stark verdichten, daß die nachfolgende Schneidkante blockiert wird. Auf diese Weise ist sichergestellt, daß nur Gewebe aus dem Zielgebiet entnommen wird. Durch die Befestigung des Mandrins am Kolben kann dieser schnell und mit einem Zug zurückgezogen und die Schneidkante der Stanzkanüle zur eigentlichen Probeentnahme freigegeben werden. Dadurch ist eine schnelle und sichere Handhabung möglich. Auch können aus locker aufgehängten Organen Proben entnommen werden, ohne daß diese ausweichenden können. Die hohe Auflösung moderner Ultraschallgeräte läßt immer häufer Organveränderungen erkennen, die einer weiteren Abklärung bedürfen. Die Entnahme von Gewebeproben mittels perkutaner Feinnadelpunktion mit der erfindungsgemäßen Kanüle ist dabei risikoarm möglich. Die scharfe Mandrinspitz überragt die Schneidkante der Kanüle um ca. 2 mm. Dabei folgt die Kanülen-Schneidkante der am äußeren Umfang verlaufenden Mandrin-Schneidkante, wodurch beide Schneidkanten eine funktionell einheitliche Schneidkante bilden. Diese funktionell einheitliche, spitze und sehr scharfe Schneidkante ermöglicht ein glattes Durchtrennen der Epidermis ohne vorherige Stichinzision. Die unmittelbar folgende Biopsiekanüle findet in der vom Mandrin vorgeschnittenen Bahn praktisch keinen Widerstand. Hat die sonographisch gut sichtbare Nadelspitz den Punktionsort erreicht, so wird der am Spritzenkolben befestigte Mandrin zurückgezogen und gleichzeitig ein Unterdruck aufgebaut. Der Mandrin bewirkt nun als Stopsonde, der das Verschleppen des aspirierten Materials in der Spritze verhindert.

Weitere vorteilhafte Maßnahmen sind in den Unteransprüchen beschrieben. Die Erfindung ist in der beiliegenden Zeichnung dargestellt und wird nachfolgend näher beschrieben; es zeigt:

Fig. 1 die Seiten ansicht einer Biopsiekanüle mit einem Schräganschliff mit Hinterschliff und einem Mandrin mit gleichsinnig orientiertem Schräganschliff mit Facettenschliff;

Fig. 2 die Rückansicht einer Biopsiekanüle nach der Fig. 1 mit Schräganschliff, Hinterschliff und Mandrin;

Fig. 3 die Vorderansicht einer Biopsiekanüle nach Fig. 1 mit einem Mandrin mit Längsbohrung und Facettenschliff;

Fig. 4 die Vorderansicht einer Biopsiekanüle nach Fig. 1 mit einer Kanüle mit Schräganschliff und einem vollen Mandrin mit asymmetrischem Facettenschliff;

Fig. 5 die Draufsicht auf die Kanüle mit Hinterschliff und den Mandrin mit Facettenschliff nach der Fig. 1;

Fig. 6 die Draufsicht auf den Mandren mit außermittiger Mandrinspitze mit Facettenschliff nach der Fig. 4;

Fig. 7 den Schnitt durch eine Biopsiekanüle nach Fig. 1, mit an dem Spritzenkolben befestigtem Mandrin;

Fig. 8 den Schnitt durch ein Biopsiebesteck nach der Fig. 7 mit Aspirationsspritze und Spritzenhalterung zur Einhandbedienung.

Das in der Zeichnung dargestellte Biopsieinstumentarium 10 besteht im wesentlichen aus einer Feinnadelbiopsie-Kanüle 11 mit einem längsverschiebbaren Mandrin 17. Die Kanüle 11 weist einen schrägen Hauptschliff 12 mit einem Facettenschliff 16 auf, der in Hinterschliff-Facetten 13 übergeht. Dadurch entsteht im Bereich des äußeren freien Endes der Kanüle 11 eine Kanülenspitze 28 mit einer sehr scharfen, spitzen Schneidkante 45. Die Schlifflänge 15 des Hauptschliffes 12 ist etwa zwei- bis viermal, vorzugsweise dreimal so groß wie der Außendurchmesser 14 der Kanüle 11. Dadurch ergibt sich ein Anschliffwinkel 23 von ca. 20—30 o, vorzugsweise von 20 o. Der Außendurchmesser 14 der Kanüle 11 beträgt 0,6 bis 0,9 mm und liegt damit im Feinnadelbereich mit auf ein Minimum reduziertem Komplikationsrisiko.

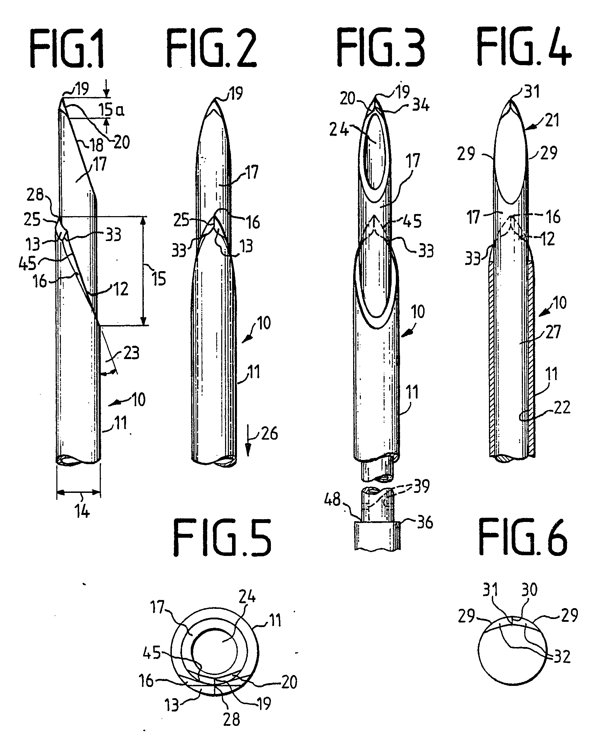

Wie in den Figuren 1 bis 5 dargestellt ist, ist die Kanüle 11 mit einem Innelumen 22 versehen, in dem ein Mandrin 17 verschiebbar angeordnet ist. Der Mandrin 17 weist einen schrägen Hauptschliff 18 auf, der mit einem von der Mandrinspitze 19 ausgehenden Anschliff 20 versehen ist. Die Schlifflänge 15a dieses Anschliffes 20 ist relativ kurz und beträgt ca. 1/5 bis 1/3 der Schlifflänge 15 des Schräganschliffs 12.

Wie die Figuren 1 und 2 zeigen, verlaufen die Hinterschliff-Facetten 13 der Kanüle 11 gegen den Anschliff 20 des Mandrins 17. Durch diese beiden Schliffe 13 und 20 entsteht eine spitze, scharfe Schneidkante 45 mit einer Längsabwinklung 25, mit der die Lederhaut leicht und richtungstreu durchstoßen werden kann. Die Hauptschliffe 12 und 18 sind dabei gleichsinnig orientiert und in ihrer Orientierung festgelegt.

Bei der in der Fig. 3 dargestellten Ausführung ist der Mandrin 17 hohl ausgebildet und mit einer Mandrin-Längsbohrung 24 versehen. Diese Längsbohrung 24 ist bei diesem Ausführungsbeispiel am Hinterende 48 des Mandrin 17 mit Querbohrungen 39 im Bereich des Spritzenkolbens 36 versehen. Durch diese Längsbohrung 24 und die Querbohrungen 39 kann das in der Aspirationsspritze 37 vorhandene Vakuum schnell an die Kanülenspitze 28 übertragen und die Probennahme unterstützt werden. Auch können dadurch Proben aus eventuell flüssigen Zielgebieten gewonnen werden, die im Ultraschall nicht oder nur sehr schwer als solche erkennbar sind. Derartige flüssige Proben können durch die Bohrungen 24 und 39 in Richtung auf das Kanülenende 26 gesogen werden oder bis in den Innenraum 47 Spritze 37 gelangen.

Der mit einem schrägen Hauptschliff 18 versehene Mandrin 17 weist im Bereich seiner Mandrinspitze 19 eine Längsabwinklung 34 auf, die ein schnelles und scharfes Durchtrennen der Epidermis erleichtert. Die Kanüle 11 ist mit dem Hauptschliff 12 mit Hinterschliff-Facetten 13 versehen und weist im Bereich der Kanülenspitze 28 eine Längsabwinklung 25 und im Bereich der Schneidkante 45 eine Schrägabwinklung 33 auf. Der Hauptschliff 12 ist asymmetrisch und die Kanülenspitze 28 außermittig gelegen.

In der Fig. 4 ist eine Biopsiekanüle 11 dargestellt, bei der der Mandrin 17 ganz aus einem Vollmaterial 27 besteht. Die Mandrinspitze 31 ist dabei mit einem asymmetrischen Trokarschliff 21 versehen. Der Mandrin 17 hat, wie in der Fig. 6 in Draufsicht dagestellt ist, zwei gebogene, schrägverlaufende Schneidkanten 29 und eine gerade, senkrechtverlaufende Schneidkante 30, die in einer außermittig gelegenen Mandrinspitze 31 zusammenlaufen. Diese in Draufsicht außermittig gelegene Mandrinspitze 31 ist gleichsinnig orientiert zu der ebenfalls außermittig gelegenen Kanülenspitze 28 und in dieser Orientierung festgelegt. Die auf die asymmetrisch angeordneten Schneidkanten 29 und 30 zulaufenden Schneidenflächen 32 können konkav gewölbt sein. Durch diese Maßnahmen können auch locker aufgehängte Organe, beispielsweise Nieren und dgl., punktiert werden, ohne daß die Gefahr besteht, daß sie der Biopsiekanüle ausweichen. Fehlpunktionen können so wirksam vermieden werden.

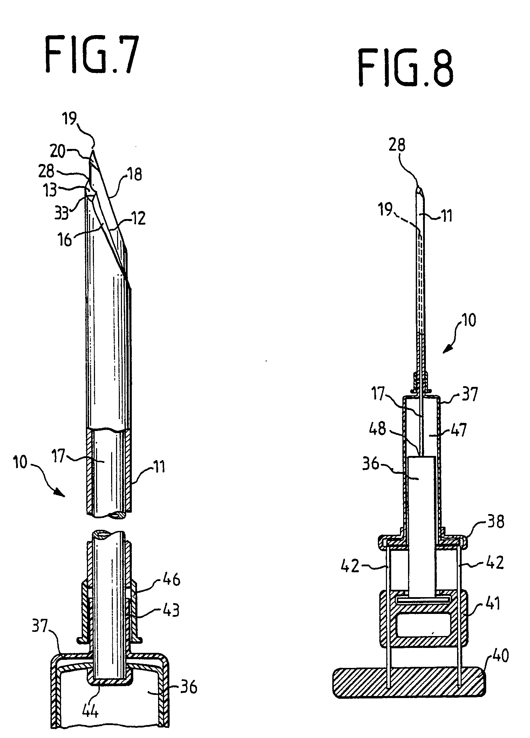

Wie die Fig. 7 zeigt, ist in der Kanüle 11 ein Mandrin 17 mit schrägen Hauptschliff 18 angeordnet, der genau in der Ebene des Hauptschliffes 12 der Kanüle 11 verläuft. Der Mandrin 17 ist im Bereich des Spritzenkonus 46 in einer Mandrinführung 43 geführt und durch eine Mandrinhalterung 44 fest mit der Kolbenstange 36 der Aspirationsspritze 37 verbunden.

Die Aspirationsspritze 37 kann, wie die Fig. 8 zeigt, in eine Spritzenhalterung 38 für Einhandbedienung adaptiert sein. Diese Spritzenhalterung 38 besteht im wesentlichen aus einem Handgriff 40 zur Handhabung der Spritze 37, einer Kolbenhalterführung 42, über die der Handgriff 40 mit der Spritze 37 verbunden ist, und einem, mit dem Kolben 36 verbundenen Kolbenhalter 41, der auf der Kolbenhalterführung 42 verschiebbar ist. Eine solche Spritzenhalterung 38 ermöglicht es, die Biopsiekanüle 11 mit nur einer Hand in das Gewebe des auf dem Monitor eines Ultraschallgerätes ausgemachten Zielgebietes zu stoßen und durch Zurückziehen des 10 Kolbens 36 und damit des Mandrins 17 bei sich unmittelbar anschließendem Vorstoßen der Kanüle 11 mit der nunmehr freigegebenen Schneidkante 45 eine Gewebeprobe zu entnehmen. Dieser Eingriff ist sehr schnell und damit relativ schmerzfrei für den Patienten durchführbar.

Zum leichteren Eindringen der Kanüle 11 ist der Mandrin 17 über die Schneidkante 45 der Kanüle 11 hinaus vorgeschoben. Dadurch wird ein glatter, gewebeschonender Stichkanal erzeugt, das benachbarte Gewebe nicht zerrissen und nicht gedehnt. Die Komplikationsrate kann dadurch erheblich gesenkt werden. Die sehr feine Kanüle 11 kann dem Mandrin 17 folgen, ohne daß sich Fremdgewebe an ihr festsetzen kann. Da diese sehr feine Kanüle 11 einen Außendurchmesser von nur ca. 0,6 bis 0,9 mm aufweist, fällt sie unter Feinnadelrisiko und kann ohne große Schmerzen und Verletzungen am Patienten eingesetzt werden.

Bezugzeichenliste

10 Biopsieinstrumentarium
11 Biopsiekanüle
12 Schräganschliff, Kanüle
13 Hinterschliff-Facette
14 Außendurchmesser
15 Schlifflänge
15a kurze Schlifflänge
16 Facettenschliff, Kanüle
17 Mandrin
18 Schräganschliff, Mandrin
19 Mandrinspitze
20 Facettenschliff, Mandrin
21 asymmetrischer Trokarschliff
22 Innenlumen
23 Schliffwinkel
24 Mandrinlängsbohrung
25 Kanülenlängsabwinklung
26 Kanülenende
27 Vollmaterial
28 Kanülenspitze
29 Schneidkante
30 Schneidkante
31 Mandrinspitze
32 Schneidfläche
33 Schrägabwinklung
34 Längsabwinklung
35 Schnittpunkt
36 Kolbenstange
37 Aspirationsspritze
38 Spritzenhalterung
39 Querbohrung
40 Handgriff
41 Kolbenhalter
42 Kolbenhalterführung
43 Mandrinführung
44 Mandrinhalterung
45 Schneidkante
46 Spritzenkonus
47 Innenraum
48 Hinterende.

**Patentansprüche**

1. Biopsiebesteck mit
einer Kanüle (11), die einen schrägen Hauptschliff (12) mit kleinem Schliffwinkel und eine umlaufende Schneidkante (45) aufweist, und
einem gegen Verdrehung zu der Kanüle (11) gesicherten, über den Kanülenhauptschliff (12) herausragenden zylindrischen Mandrin (17), der eine Mandrinspitze (19) aufweist,
wobei die Kanüle (11) zur Probenentnahme über den Mandrin hinaus vorschiebbar ist, dadurch gekennzeichnet, daß der Mandrin (17) einen schrägen Hauptschliff (18) aufweist, dessen Schliffwinkel und Orientierung derjenigen des Hauptschliffs (12) der Kanüle (11) entsprechen,
daß die Mandrinspitze (19) durch einen Anschliff (20) des Mandrin-Hauptschliffs (18) gebildet ist,
und daß die Kanüle (11) eine Kanülenspitze (28) aufweist, die von Hinterschliff-Facetten (13) der Schneidkante (45) gebildet ist, welche bis an das Innenlumen (22) der Kanüle (11) herangeschliffen sind.

2. Biopsiebesteck nach Anspruch 1, dadurch gekennzeichnet, daß das Hinterende (48) des Mandrins (17) mit der Kolbenstange (36) einer Aspirationsspritze (37), auf welche die Kanüle (11) montiert ist, verbunden ist.

3. Biopsiebesteck nach Anspruch 1 oder 2, dadurch gekennzeichnet, daß das Hinterende (48) des Mandrins (17) mit einem Griff versehen ist, welcher zum Griff der Kanüle (11) gegen Verdrehung gesichert ist.

4. Biopsiebesteck nach einem der Ansprüche 1 bis 3, dadurch gekennzeichnet, daß der Anschliff (20) des Mandrins (17) eine Schlifflänge (15a) aufweist, deren Anteil an der Länge des Mandrin-Hauptschliffs (18) kleiner ist als 50%.

5. Biopsiebesteck nach einem der Ansprüche 1 bis 4, dadurch gekennzeichnet, daß der Hauptschliff (12) der Kanüle (11) einen Schliffwinkel von 20° bis 30°, vorzugsweise von 25° zur Längsachse aufweist.

6. Biopsiebesteck nach einem der Ansprüche 1 bis 5, dadurch gekennzeichnet, daß der Mandrin (17) als Hohlmandrin ausgebildet ist.

7. Biopsiebesteck nach einem der Ansprüche 2 bis 6, dadurch gekennzeichnet, daß der als Hohlmandrin ausgebildete Mandrin (17) im Bereich des Spritzenkolbens (36) über Querbohrungen (39) mit dem Innenraum (47) der Aspirationsspritze (37) verbunden ist.

8. Biopsiebesteck nach einem der Ansprüche 1 bis 5, dadurch gekennzeichnet, daß der Mandrin (17) als Vollmandrin ausgebildet ist.

9. Biopsiebesteck nach einem der Ansprüche 1 bis 8, dadurch gekennzeichnet, daß der Hauptschliff (18) des Mandrins (17) als asymmetrischer Trokarschliff (21) ausgebildet ist.

**Revendications**

1. Nécessaire à biopsie comprenant une canule (11) présentant un tranchant principal oblique (12) à faible angle de tranchant et un bord tranchant (45) sur tout le pourtour, et

un mandrin cylindrique (17), présentant une pointe de mandrin (19), en saillie par rapport au tranchant principal (12) de la canule, bloqué en rotation par rapport à la canule (11),

la canule (11) pouvant, pour le prélèvement d'échantillons, être glissée vers l'avant, par dessus le mandrin,

caractérisé en ce que le mandrin (17) présente un tranchant principal oblique (18) dont l'angle de tranchant et l'orientation correspondant à ceux du tranchant principal (12) de la canule (11),

que la pointe de mandrin (19) est formée par une coupe polie (20) du tranchant principal (18) du mandrin,

et que la canule (11) présente une pointe de canule (28) formée par des facettes arrières polies (13) du bord tranchant (45) réalisées par polissage jusqu'au passage intérieur (22) de la canule (11).

2. Nécessaire à biopsie suivant la revendication 1, caractérisé en ce que l'extrémité arrière (48) du mandrin (17) est reliée à la tige de piston (36) d'une seringue d'aspiration (37) sur laquelle est montée la canule (11).

3. Nécessaire à biopsie suivant la revendication 1 ou 2, caractérisé en ce que l'extrémité arrière (48) du mandrin (17) est pourvue d'un manche bloqué en rotation par rapport au manche de la canule (11).

4. Nécessaire à biopsie suivant l'une des revendications 1 à 3, caractérisé en ce que la coupe polie (20) du mandrin (17) présente une longueur de tranchant (15a) dont la part dans la longueur du tranchant principal (18) du mandrin est inférieure à 50%.

5. Nécessaire à biopsie suivant l'une des revendications 1 à 4, caractérisé en ce que le tranchant principal (12) de la canule (11) présente un angle de tranchant de 20° à 30°, de préférence de 25° par rapport à l'axe longitudinal.

6. Nécessaire à biopsie suivant l'une des revendications 1 à 5, caractérisé en ce que le mandrin (17) se présente sous forme de mandrin creux.

7. Nécessaire à biopsie suivant l'une des revendications 2 à 6, caractérisé en ec que le mandrin (17) se présentant sous forme de mandrin creux est en communication, à l'endroit du piston de seringue (36), par des orifices transversaux (39), avec la chambre intérieure (47) de la seringue d'aspiration (37).

8. Nécessaire à biopsie suivant l'une des revendications 1 à 5, caractérisé en ce que le mandrin (17) se présente sous forme de mandrin plein.

9. Nécessaire à biopsie suivant l'une des revendications 1 à 8, caractérisé en ce que le tranchant principal (18) du mandrin (17) se présente sous forme de tranchant de trocart asymétrique (21).

**Claims**

1 Biopsy set comprising
a cannula (11) having a ground main bevel (12) with small bevel angle, and a surrounding cutting edge (45), and
a cylindrical stylet (17) secured against rotation with respect to the cannula (11) and protruding beyond the ground main bevel (12) of the cannula, said stylet (17) having a stylet tip (19),
the cannula (11) being adapted to be advanced beyond the stylet for collecting a sample, characterized in
that the stylet (17) is provided with a ground main bevel (18) with its bevel angle and orientation corresponding to those of the ground main bevel (12) of the cannula (11),
that the stylet tip (19) is formed by a ground portion (20) ground to the ground main bevel (18) of the stylet, and
that the cannula (11) has a cannula tip (28) being formed by ground rear facettes (13) of the cutting edge (45), which are ground up to the inner lumen (22) of the cannula (11).

2. Biopsy set according to claim 1, characterized in that the rear end (48) of the stylet (17) is connected to the piston rod (36) of an aspiration syringe (37) having the cannula (11) mounted thereon.

3. Biopsy set according to claim 1 or 2, characterized in that the rear end (48) of the stylet (17) is provided with a handle secured against rotation with respect to the handle of the cannula (11).

4 Biopsy set according to any one of claims 1 to 3, characterized in that the ground portion (20) of the stylet (17) has a grinding length (15a) constituting a portion of less than 50% of the length of the ground main bevel (18) of the stylet.

5. Biopsy set according to any one of claims 1 to 4, characterized in that the ground main bevel (12) of the cannula (11) has a bevel angle of 20° to 30°, preferably 25°, with respect to the longitudinal axis thereof.

6. Biopsy set according to any one of claims 1 to 5, characterized in that the stylet (17) is provided as a hollow stylet.

7. Biopsy set according to any one of claims 2 to 6, characterized in that, in the area of the syringe piston (36), the stylet (17) provided as a hollow stylet is connected to the inner space (47) of the aspiration syringe (37) via transverse bores (39).

8. Biopsy set according to any one of claims 1 to 5, characterized in that the stylet (17) is provided as a full stylet.

9. Biopsy set according to any one of claims 1 to 8, characterized in that the ground main bevel (18) of the stylet (17) is formed as an asymmetrical trocar bevel (21).

# FIG.1

# FIG.2

# FIG.3

# FIG.4

# FIG.5

# FIG.6

# FIG.7

# FIG.8